# EUROPÄISCHE PATENTANMELDUNG

(11) **EP 2 742 917 A1**
(43) Veröffentlichungstag der Anmeldung: **18.06.2014**
(21) Anmeldenummer: 13194242.7
(22) Anmeldetag: 25.11.2013
(51) Int. Cl.: A61F 2/95

(54) **Vorrichtung und Verfahren zum Crimpen eines Implantats**

(30) Priorität: 13.12.2012 US 201261736555 P
(71) Anmelder: Biotronik AG, 8180 Bülach (CH)
(72) Erfinder: Fargahi, Amir, 8180 Bülach (CH)
(74) Vertreter: Galander, Marcus

(57) **Zusammenfassung**

Die vorliegende Erfindung betrifft Vorrichtung zum Crimpen eines Implantats (30), insbesondere einer intraluminalen Endoprothese, welches zumindest über einen Teil seiner Länge entweder einen komprimierten Zustand oder einen expandierten Zustand einnehmen kann. Eine einfach handhabbare, kleinbauende und kostengünstige Crimpvorrichtung wird erfindungsgemäß dadurch realisiert, dass eine Walzenanordnung mit mindestens drei Walzen (1, 2, 3) zum Einspannen des Implantats (30) zwischen diesen vorgesehen ist, wobei mindestens eine Walze (1) der Walzenanordnung in ihrem Abstand zu den jeweils anderen mindestens zwei Walzen (2, 3) derart verringerbar ist, dass ein zwischen den Walzen (1, 2, 3) eingespanntes Implantat (30) wenigstens über einen Teil seiner Länge von dem expandierten Zustand in den komprimierten Zustand überführbar ist. Die Erfindung betrifft ferner ein entsprechendes Verfahren zum Crimpen eines Implantats.

## Beschreibung

Die vorliegende Erfindung betrifft eine Vorrichtung zum Crimpen eines Implantats, insbesondere einer intraluminalen Endoprothese, und ein entsprechendes Verfahren.

Medizinische Implantate, insbesondere intraluminale Endoprothesen, für die unterschiedlichsten Anwendungen sind in großer Vielfalt aus dem Stand der Technik bekannt. Als Implantate im Sinne der vorliegenden Erfindung sind endovaskuläre Prothesen oder sonstige Endoprothesen, beispielsweise Stents (Gefäß-Stents (vaskuläre Stents, einschließlich Stents für die Anwendung im Bereich des Herzens und Herzklappenstents, z.B. Mitralklappen-Stent, Pulmonalklappenstent) und Gallengang-Stents), Endoprothesen zum Verschließen von persistierenden Foramen ovale (PFO), Stentgrafts zur Behandlung von Aneurysmen, Endoprothesen zum Verschließen eines ASD (Vorhofscheidewanddefekt, atrial septal defect) sowie Prothesen im Bereich des Hart- und Weichgewebes zu verstehen. Ein derartiges Implantat wird häufig mittels eines Katheters in das zu behandelnde Organ oder Gefäß eingesetzt.

Stents und andere Implantate weisen in vielen Fällen eine durchbrochene hohlzylinderförmige (rohrförmige) und/oder hohlkegelförmige Grundstruktur auf, die an beiden Längsenden offen ist, wobei die Grundstruktur häufig aus einer Vielzahl von Stegen zusammengesetzt ist. In einer derartige Grundstruktur können z.B. bei einem Herzklappenstent auf der Innenseite Klappensegel, beispielsweise drei Klappensegel, angeordnet sein, welche die Herzklappe ausbilden und aus einem Kunststoff oder einem biologischen Material, z.B. Schweine-Pericard, bestehen können. In diesem Fall trägt der Stent die Herzklappe und verankert diese im Herzen.

Stents und andere Implantate nehmen üblicherweise zwei Zustände ein, nämlich einen komprimierten Zustand mit einem kleinen Durchmesser und einen expandierten Zustand mit einem größeren Durchmesser. Im komprimierten Zustand kann das Implantat mittels eines Katheters in das zu behandelnde Gefäß oder Organ durch enge Gefäße hindurch eingeführt und an der zu behandelnden Stelle positioniert werden. Hierfür wird das Implantat gecrimpt und dadurch wenigstens über einen Teil seiner Länge von dem expandierten Zustand mit größerem Durchmesser in den komprimierten Zustand mit kleinerem Durchmesser überführt. Am Ort der Behandlung wird das Implantat dann beispielsweise mittels des Ballons des Katheters dilatiert und nimmt dann den wieder den expandierten Zustand ein, in dem das Implantat in dem Gefäß oder Organ verbleibt und dort festgelegt ist, nachdem der Katheter aus dem Körper des Behandelten entfernt wurde. Alternativ nimmt ein Implantat in dem Fall, in dem seine Grundstruktur aus einem selbstexpandierenden Material (z.B. Nitinol) besteht, den komprimierten Zustand durch Komprimierung unterhalb der Übergangstemperatur und den expandierten Zustand oberhalb der Übergangstemperatur ein.

Aus der Druckschrift US 8,029,564 B2 ist eine Herzklappenprothese und eine Umlenkeinrichtung bekannt. Das System beinhaltet ferner ein Band, das durch die freien Enden der Stentstangen, welche die Herzklappe tragen, hindurch geführt wird. Mittels dieses Bandes können die Stentstangen nach innen umgebogen werden, um einen geschlossenen Zustand zu erreichen. Dieses System eignet sich jedoch nicht dafür, nichtinvasiv mittels eines Katheters zu der behandelnden Stelle transferiert zu werden, da es im Fußbereich zu voluminös ist.

Aus der Druckschrift US 2011/0056064 A1 ist ein Crimpwerkzeug bekannt, das sehr kostenintensiv in der Herstellung ist, da es einen komplexen Aufbau mit einer Vielzahl von präzise zu fertigenden Teilen aufweist, welche sich synchron bewegen müssen. Das Crimpwerkzeug setzt sich insbesondere aus einer Vielzahl von Riegeln zusammen, die entlang eines Kreisumfangs nebeneinander und um eine quer zum jeweiligen Riegel verlaufende Achse drehbar angeordnet sind. In die zwischen den Enden der Riegel ausgebildete Öffnung ist das zu crimpende Implantat aufnehmbar. Durch die Bewegung eines Hebels werden Führungpins, wobei jeweils ein Pin an einem vorderen Ende eines Riegels angeordnet ist, in einem Langloch verschoben, dass der Radius der zwischen den Riegeln ausgebildeten Öffnung verringert oder vergrößert wird. Für das Crimpen eines Herzklappenstents werden die Pins derart verschoben, dass der Radius der Öffnung verkleinert wird. Bei diesem Werkzeug ist der Stent während des Crimpvorgangs vollständig durch dieses überdeckt, da die Riegel und die Pins zwischen zwei Platten angeordnet sind und das Werkzeug daher in Längsrichtung des Stents eine große Ausdehnung und ein vergleichsweise hohes Gewicht aufweist. Aus diesem Grund kann das Implantat während des Crimpens visuell nicht überwacht werden. Ferner ist nicht auszuschließen, dass die Riegel bei der Verkleinerung des Öffnungs-Radius' überlappen, so dass das Risiko einer Verletzung des Implantats sowie der an dem Implantat angeordneten Gewebeteile hoch ist. Die große Masse des Werkzeugs führt ferner dazu, dass dieses nur mit großem Aufwand unter die Übergangstemperatur bringbar ist, was notwendig ist, wenn das Implantat aus einem selbst expandierenden Material besteht.

Die Druckschrift EP 2 229 921 A1 offenbart eine Vorrichtung zum Crimpen, welche eine Vielzahl von entlang einer Zylinder-Mantellinie zwischen zwei beabstandeten Ringen gespannten Drähten aufweist. Wird einer dieser Ringe relativ zu dem zweiten Ring um eine Achse parallel zu diesen Mantellinien gedreht, so werden die Drähte zu der gemeinsamen Achse verdreht, so dass diese eine Urglas-ähnliche geometrische Fläche ähnlich zu einem Rotationshyperboloide ausbilden. Die durch den Abschnitt mit dem kleinsten Durchmesser definierte Öffnung, welche diese Drähte zwischen sich ausbilden, wird bei zunehmender Verdrehung der Ringe verkleinert oder vergrößert. In diese Öffnung kann ein Implantat angeordnet und bei Verkleinerung der Öffnung gecrimpt werden. Diese bekannte Vorrichtung ist ebenfalls vergleichsweise kompliziert aufgebaut, so dass deren Montage schwierig ist. Das Implantat wird während des Crimpens durch die Drähte der Vorrichtung überdeckt, so dass der Crimpprozesses auch bei dieser bekannten Vorrichtung nicht visuell überwacht werden kann. Die Gefahr, dass eine Überlappung von Segmenten des Implantats während des Crimpens erfolgt, ist hoch. Nachteilhaft ist ferner, dass die Drähte an den beiden Enden, an denen die Drähte an den gegenüber liegenden Ringen befestigt sind, stärker gespannt werden als in ihrem mittleren Bereich. Dies führt zu einer ungleichmäßigen Verteilung der Kompressionskraft auf das Implantat. Aufgrund der vielen Drähte und der notwendigen großen Ringe an deren Enden ist die bekannte Vorrichtung vergleichsweise groß und schwer.

Die Aufgabe besteht somit darin, eine Vorrichtung zum Crimpen eines derartigen Implantats zu schaffen, welche weniger kompliziert aufgebaut sowie kleiner und handlicher gebaut ist. Entsprechend soll ein Verfahren zum Crimpen angegeben werden, welches einfacher durchführbar ist.

Die obige Aufgabe wird durch eine Vorrichtung mit den Merkmalen des Anspruchs 1 gelöst.

Insbesondere weist die erfindungsgemäße Vorrichtung eine Walzenanordnung mit mindestens drei Walzen zum Einspannen des Implantats zwischen diesen auf, wobei mindestens eine Walze der Walzenanordnung in ihrem Abstand zu den jeweils anderen mindestens zwei Walzen derart verringerbar ist, dass ein zwischen den Walzen eingespanntes Implantat wenigstens über einen Teil seiner Länge von dem expandierten Zustand in den komprimierten Zustand überführbar ist.

Vorzugsweise ist jede Walze der Walzenanordnung um ihre Längsachse drehbar in einem C-förmigen Träger befestigt, wobei die Längsachsen der Walzen vorzugsweise parallel zueinander und besonders bevorzugt parallel zu der Längsachse des zu crimpenden Implantats verlaufen, wenn dieses zwischen den Walzen eingespannt ist.

Die oben angegebenen Vorrichtung ist vergleichsweise einfach sowie kleinbauend und aufgebaut und erlaubt einen Zugang des zu crimpenden Implantats von außen zu Überwachungszwecken, da der C-förmige Träger das Implantat nicht vollständig umschließt. Ferner hat die Anordnung des Implantats zwischen den mindestens drei Walzen den Vorteil, dass das vorzugsweise hohlzylinder- oder hohlkegel-förmige Implantat mit seiner Mantelfläche auf den Mantelflächen der Walzen abrollen kann. Hierdurch wird das Risiko einer Verletzung des Implantat beziehungsweise des an der Grundstruktur des Implantats angeordneten Gewebes deutlich verringert. Das Gehäuse der Vorrichtung, welches die Walzen aufnimmt und vorzugsweise C-förmig gestaltet ist, kann aus Kunststoff gespritzt werden, so dass eine weitere Gewichtsreduzierung und eine weitere Vereinfachung der Handhabung erreicht wird. Als Achsen, Walzen beziehungsweise weiter unten beschriebene Bauteile, welche zur Verringerung des Abstands der Walzen dienen, können Standard-Bauteile eingesetzt werden, welche sehr kostengünstig sind. Zudem wird die Kompressionskraft durch die Walzen homogen und gleichmäßig über die gesamte Länge des zu crimpenden Bereichs verteilt auf das Implantat übertragen. Da für das Gehäuse Kunststoff eingesetzt werden kann, ergeben sich zudem Vorteile hinsichtlich des Gewichts der Vorrichtung, so dass auch deren Handhabung erleichtert wird.

Als Abstand der Walzen der Walzenanordnung zueinander wird die Entfernung zwischen deren Längsachsen definiert, wenn die Walzen quer zu ihrer Längsachse geschnitten werden.

Wie oben bereits ausgeführt wurde, sind die Walzen der Walzenanordnung vorzugsweise in einem C-förmigen Träger befestigt, welcher besonders bevorzugt 2-teilig ausgebildet ist. Um den Abstand zwischen den Walzen verändern zu können, ist der erste Teil des Trägers, an welchem vorzugsweise lediglich eine Walze befestigt ist, verschieblich entlang einer an den zweiten Teil des Trägers drehbar befestigten Spindel angeordnet. Der zweite Teil des Trägers weist vorzugsweise zwei Walzen auf. Der erste Trägerteil sieht zudem einen Innengewindeabschnitt vor, der mit dem Außengewinde der Spindel des zweiten Trägerteils zusammenwirkt und in dieses eingreift. Beim Drehen der Spindel verschiebt sich der erste Trägerteil bei Verringerung des Abstands zwischen den Walzen dabei gegen die Kraft einer zwischen dem ersten Trägerteil und dem zweiten Trägerteil gelagerten Druckfeder.

Hierbei ist von Vorteil, wenn die Einrichtung zur Verringerung des Abstands der Walzen zueinander seitlich zu der Walzenanordnung, d.h. quer zu den Längsachsen neben den Walzen, welche den Einspannabschnitt ausbilden, angeordnet ist, da hierdurch die Ausdehnung der Vorrichtung in Längsrichtung nicht vergrößert wird. Die Handhabbarkeit der erfindungsgemäßen Vorrichtung wird dadurch verbessert. Bei dem oben angegebenen Stand der Technik sind die Elemente, welche zur Verringerung eines Durchmessers dienen, vor oder hinter dem Einspannabschnitt für das Implantat in Richtung der Längsachse angeordnet.

In einem weiteren bevorzugten Ausführungsbeispiel weist mindestens eine Walze der Walzenanordnung eine Mantelfläche auf, welche Teflon enthält. Hierdurch wird die Reibung zwischen den jeweiligen Walzen und dem Implantat verringert, so dass das Risiko für eine Beschädigung des Implantats weiter verringert wird. Vorzugsweise weisen alle Walzen der Walzenanordnung auf der Oberfläche ihrer Mantelfläche eine Teflon-Beschichtung auf.

Es ist weiterhin bevorzugt, dass die Vorrichtung um das Implantat bzw. relativ zu diesem drehbar ausgebildet ist, insbesondere ist die Vorrichtung um das Implantat drehbar ausgebildet. Die Abstandsänderung kann auch automatisch erfolgen.

Insbesondere für das Crimpen von Implantaten mit einer Grundstruktur, welche ein selbst expandierendem Material enthält, ist es von Vorteil, wenn die erfindungsgemäße Vorrichtung in ein Kühlmittel einbringbar ist. Dieses Kühlmittel kühlt das Implantat auf eine Temperatur unterhalb der Übergangstemperatur ab. Da die erfindungsgemäße Vorrichtung vergleichsweise kleinbauend ist, muss lediglich ein vergleichsweise kleiner Behälter mit dem Kühlmittel bereitgestellt werden, in den die erfindungsgemäße Vorrichtung eingetaucht werden kann. Die Größe des Kühlmittelbehälters sollte dabei so gewählt werden, dass die erfindungsgemäße Vorrichtung hinein passt. Der Behälter sollte zudem sterilisierbar sein.

Die obige Aufgabe wird ferner durch ein einfaches Verfahren zum Crimpen eines Implantats mit den Merkmalen des Anspruchs 7 gelöst.

Hierbei werden insbesondere die folgenden Schritte durchgeführt. Zunächst wird das zunächst im expandierten Zustand vorliegende Implantat mit wenigstens einem Teil seiner Länge in einer zwischen mindestens drei Walzen einer Walzenanordnung ausgebildeten Öffnung angeordnet. Dies bedeutet, dass zunächst beispielsweise eine dieser drei Walzen relativ zu den mindestens zwei übrigen Walzen derart verschoben wird, dass die dazwischen vorhandene Öffnung deutlich größer als der Außendurchmesser des Implantats im expandierten Zustand ist. Danach wird das Implantat in der Öffnung platziert und der Abstand mindestens einer Walze zu den mindestens zwei anderen Walzen der Walzenanordnung verringert, so dass zunächst die mindestens drei Walzen eng an der Außenseite des Implantats anliegen und dieses dadurch zwischen sich einspannen. Danach wird der Abstand mindestens einer Walze zu den mindestens zwei weiteren Walzen weiter derart, vorzugsweise schrittweise verringert, dass das zwischen den Walzen angespannte Implantat wenigstens über ein Teil seiner Länge von dem expandierten Zustand in den komprimierten Zustand überführt wird. Dieser im vorangegangenen Satz beschriebene Schritt wird im Folgenden auch als Crimpschritt bezeichnet. Die oben beschriebenen Vorteile der erfindungsgemäßen Vorrichtung treffen auch für das erfindungsgemäße Verfahren zu, das zudem einfach und mit geringem Zeitaufwand durchführbar ist.

Es ist ferner von Vorteil, wenn die gesamte Vorrichtung, welche das Verfahren durchführt, während der Überführung des Implantats in den komprimierten Zustand relativ zu dem Implantat gedreht wird, um den Crimpprozess über den gesamten Umfang des Implantats durchzuführen. Vorzugsweise erfolgt das Drehen der Vorrichtung relativ zu dem Implantat schrittweise abwechselnd mit der Verringerung des Abstands der mindestens einen Walze zu den mindestens zwei anderen Walzen.

Insbesondere für das Crimpen von Implantaten enthaltend selbst expandierendes Material wie Nitinol ist es von Vorteil, wenn die Vorrichtung zum Crimpen vor der Überführung des Implantats in den komprimierten Zustand in ein vorzugsweise flüssiges Kühlmittel eingebracht wird und während des Crimpschrittes in dem Kühlmittel verbleibt.

Um das gecrimpte Implantat über einen gewissen Zeitraum bis zur Einbringung in den Körper eines Patienten im komprimierten Zustand zu halten, wird in einem bevorzugten Ausführungsbeispiel nach dem Crimpen über den Teil der Länge des Implantats, welcher in den komprimierten Zustand überführt wurde, ein Schlauch angeordnet.

Beispielsweise wird das Implantat daher zunächst mit einem Teil seiner Länge, z.B. einem proximalen Ende, in die Vorrichtung geschoben und das andere Ende mit den Fingern festgehalten. Danach wird der Abstand zwischen den Walzen so weit verringert, dass das Implantat zwischen den Walzen eingespannt ist. Anschließend wird das Werkzeug samt Implantat in das Kühlmittel getaucht und beispielsweise auf 0°C abgekühlt, damit das z.B. Nitinol umfassende Material des Implantats keine Kräfte mehr zeigt. Nun wird die erfindungsgemäße Vorrichtung gedreht, anschließend der Abstand zwischen den Walzen regelmäßig und Schritt für Schritt reduziert (z.B. vollführt die Vorrichtung eine volle Umdrehung, danach wird der Walzenabstand um 5 mm reduziert, dann wird eine weitere volle Umdrehung der Vorrichtung um das eingespannte und festgehaltene Implantat durchgeführt und anschließend wieder eine Reduktion des Walzenabstands durchgeführt, usw.) bis der entsprechende Teil des Implantats gecrimpt ist. Nun wird die Vorrichtung geöffnet, d.h. der Walzenabstand vergrößert, und der Außenschlauch über den gecrimpten Teil der Prothese geschoben. Nun wird der gecrimpte und im Außenschlauch geladene Teil des Implantats festgehalten und der übrige Teil des Implantats (d.h. der noch nicht gecrimpte) auf einen geringeren Durchmesser analog zu dem obigen Verfahren überführt.

Weitere Ziele, Merkmale, Vorteile und Anwendungsmöglichkeiten der Erfindung ergeben sich aus der nachfolgenden Beschreibung von Ausführungsbeispielen zu der Erfindung anhand der Figuren. Dabei bilden alle beschriebenen und/oder bildlich dargestellten Merkmale für sich oder in beliebiger Kombination den Gegenstand der vorliegenden Erfindung, auch unabhängig von ihrer Zusammenfassung in den einzelnen Ansprüchen oder deren Rückbeziehung.

Es zeigen schematisch:
- Fig. 1: ein erstes Ausführungsbeispiel einer erfindungsgemäßen Vorrichtung mit einem zu crimpenden Implantat, jeweils in einer Ansicht von der Seite,
- Fig. 2: ein zweites Ausführungsbeispiel einer erfindungsgemäßen Vorrichtung in einer Ansicht von der Seite,
- Fig. 3: ein drittes Ausführungsbeispiel einer erfindungsgemäßen Vorrichtung im Querschnitt und
- Fig. 4 und 5: die Anordnung der Walzen und des Implantats in den Ausführungsbeispielen 1 bis 3 im Querschnitt.

Die Figuren zeigen drei Ausführungsbeispiele schematisch und vereinfacht und stellen insbesondere die Details dar, die wichtig sind, um die Erfindung zu verstehen. Für die Erfindung unbedeutende Details wurden teilweise weggelassen. Weiterhin bedeutet die Bezeichnung "distales Ende" im Zusammenhang mit der vorliegenden Erfindung das Ende des Implantats, das während dem Einbringen des Implantats in den Körper von dem behandelnden Arzt weg zeigt, während das "proximale Ende" zu der z.B. einen Katheter bedienenden Person hin zeigt.

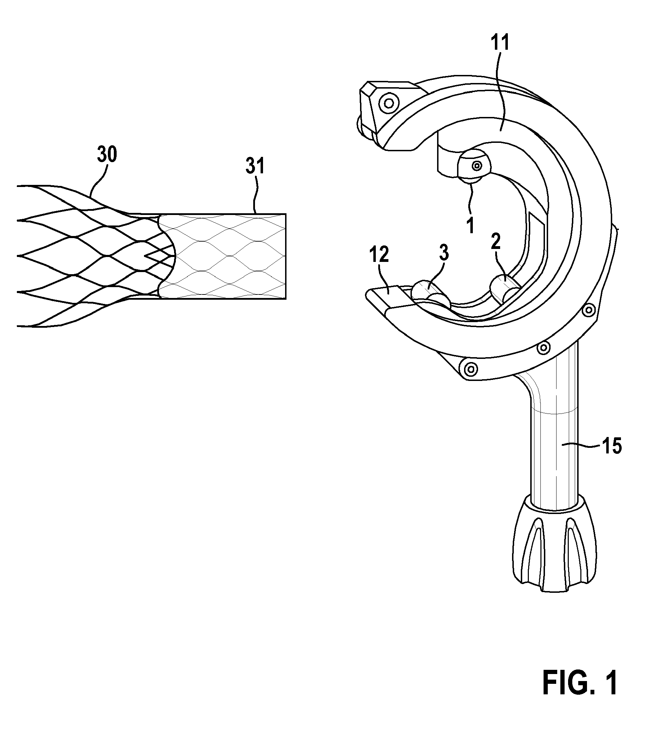

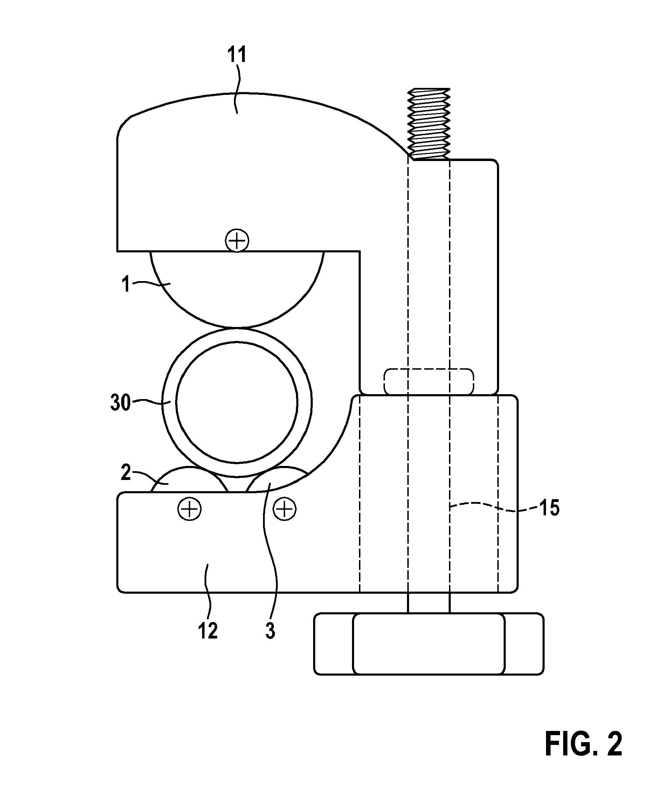

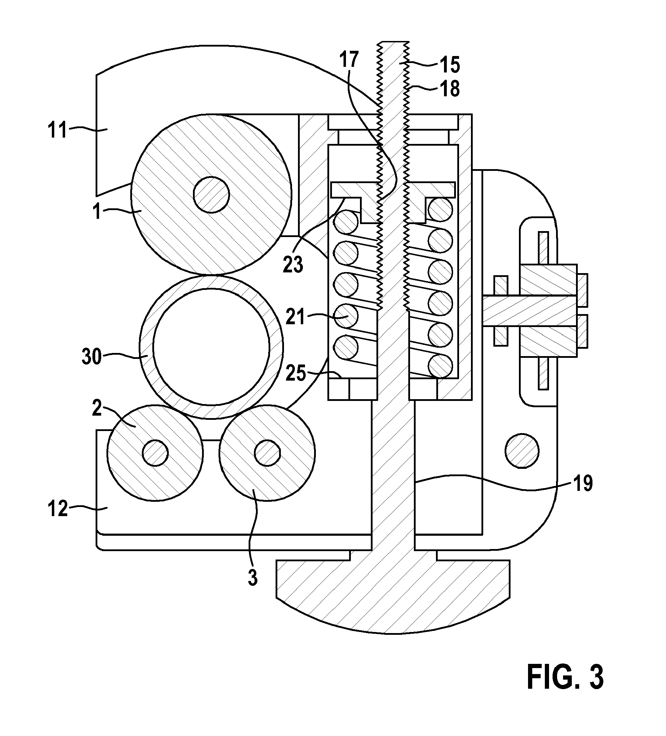

Die in den Figuren 1 bis 3 dargestellten Ausführungsbeispiele der erfindungsgemäßen Vorrichtung unterscheiden sich im Wesentlichen lediglich hinsichtlich der äußeren Gestaltung. Der für das Crimpen verantwortliche wesentliche technische Aufbau ist für alle drei Ausführungsbeispiele gleich, so dass die Vorrichtung im Folgenden insbesondere in Bezug auf Figur 3 erläutert wird, welche die Mechanik am deutlichsten zeigt. Die entsprechenden Bauteile des ersten und zweiten Ausführungsbeispiels sind mit den hinsichtlich Figur 3 gleichen Bezugszeichen versehen.

Die erfindungsgemäße Vorrichtung weist eine Walzenanordnung mit einer oberen, im Durchmesser etwas größeren Walze 1 und zwei unteren, im Durchmesser etwas kleineren Walzen 2 und 3 auf. Die erste Walze 1 ist drehbar an einem oberen, ersten Teil 11 eines C-förmigen Trägers befestigt. Die drehbare zweite Walze 2 und die dritte Walze 3 sind in einem Abstand, der größer ist als die Summe ihrer Radien, an einem zweiten Teil 12 eines C-förmigen Trägers angeordnet. Der erste Teil 11 des Trägers ist dabei entlang einer Spindel 15 gegenüber dem zweiten Teil 12 des Trägers in eine Richtung quer zu den Längsachsen der Walzen 1, 2, 3 verschieblich angeordnet. Der erste Teil 11 des Trägers weist an einer durchgehenden Öffnung 17 ein Innengewinde auf, das mit einem Außengewinde 18 der Spindel 15 zusammenwirkt und in dieses eingreift. Die Spindel 15 ist in dem zweiten Teil 12 des Trägers in einer durchgehenden Öffnung 19 drehbar gelagert. Ferner ist eine Druckfeder 21 vorgesehen, welche beispielsweise als Spiralfeder ausgebildet ist und sich zwischen einer ringförmigen Abstützfläche 23 des ersten Teils 11 des Trägers und einer ringförmigen Abstützfläche 25 des zweiten Teils des Trägers abstützt. Die Druckfeder 21, welche die Spindel 15 in ihrer durchgehenden Öffnung aufnimmt, ist bestrebt den ersten Teil 11 und den zweiten Teil 12 des Trägers auseinander zu drücken.

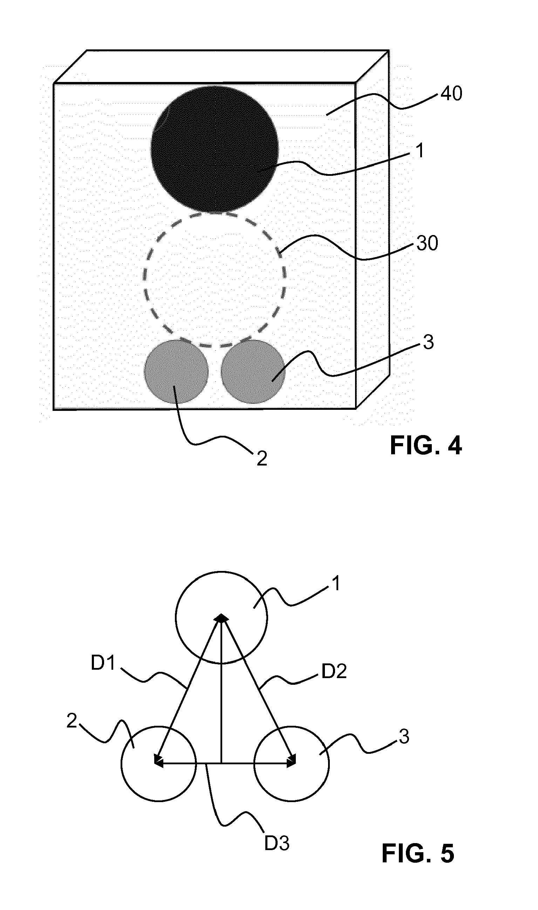

Durch Drehung der Spindel 15 um ihre Längsachse wird das Innengewinde der Öffnung 17 des ersten Teils 11 des Trägers entlang des Außengewindes 18 der Spindel 15 bewegt, und zwar gemäß der Darstellung in Figur 3 nach oben mit Unterstützung durch die Kraft der Druckfeder 21 oder nach unten entgegen der Kraft der Druckfeder 21. Hierdurch wird der in Figur 5 gezeigte Abstand D1 zwischen der ersten Walze 1 und der zweiten Walze 2 beziehungsweise der Abstand D2 zwischen der ersten Walze 1 und der dritten Walze 3 vergrößert beziehungsweise verringert. Der Abstand D3 zwischen der zweiten Walze 2 und der dritten Walze 3 wird bei diesem Beispiel hierdurch nicht verändert.

In einem bevorzugten Ausführungsbeispiel weisen die Mantelflächen der Walzen 1, 2 und 3 eine die Reibung reduzierende Beschichtung, z.B. eine Teflonbeschichtung, zur Verringerung der Reibung zwischen Implantat und Walzen auf.

Um ein Implantat, beispielsweise ein Herzklappenstent, zu crimpen, der im expandierten Zustand vorliegt, wird zunächst die erfindungsgemäße Vorrichtung ganz geöffnet. Dies bedeutet, dass die Spindel 15 solange gedreht wird bis der maximale Abstand zwischen den Walzen 1,2 und 3 erreicht wird. Gegebenenfalls wird dann die Vorrichtung im Kühlmittel 40, beispielsweise in 0°C kaltem Wasser, abgekühlt (siehe Figur 4). Der Abkühlschritt kann gegebenenfalls auch vor der Öffnung der Vorrichtung erfolgen.

Danach wird das Implantat 30, vorzugsweise mit seinem dem distalen Ende 31 gegenüber liegenden proximalen Ende zwischen die Walzen 1,2 und 3 derart eingelegt, dass die Längsachse des Implantats 30 in etwa parallel zu den Längsachsen der Walzen 1,2 und 3 verläuft, und zwischen den Walzen 1, 2, 3 eingespannt. Das distale Ende 31 des Implantats, das außerhalb der Crimpzange liegen soll, wird von Hand fest gehalten. Es kann nicht gedreht werden. Nun wird die Vorrichtung um das Implantat 30 gedreht, beispielsweise um eine Umdrehung. Anschließend wird die Spindel 15 derart gedreht, dass der Abstand zwischen der Walze 1 und den Walzen 2 und 3 schrittweise reduziert wird, beispielsweise um jeweils 5 mm. Anschließend wird die Vorrichtung wieder um eine Umdrehung um das Implantat 30 gedreht. Nun wird der Abstand zwischen den Walzen 1, 2 und 3 weiter reduziert und es folgt eine weitere Drehung der Vorrichtung usw. Hierdurch wird das Implantat, vorzugsweise zunächst an seinem proximalen Ende, gecrimpt. Dieser Crimpvorgang kann in einem bevorzugten Ausführungsbeispiel in einem, in einem entsprechend Behälter angeordneten Kühlmittel 40 (siehe Figur 4), beispielsweise in 0°C kaltem Wasser, durchgeführt werden.

Nach Beendigung des ersten Crimpvorgangs wird mittels der Spindel 15 der Abstand zwischen den Walzen 1, 2 und 3 ein klein wenig vergrößert, so dass ein Schlauch über den gecrimpten proximalen Abschnitt des Implantats geschoben werden kann, um den komprimierten Zustand zu fixieren. Danach wird das proximale Ende des Implantats, das bereits unter dem Außenschlauch vom Katheter liegt, fest gehalten und der verbleibende Abschnitt des Implantats (im vorliegenden Beispiel das distale Ende 31) gecrimpt und der Schlauch darüber geschoben. Anschließend wird das gecrimpte und im Schlauch geladene Implantat 30 aus der Vorrichtung entfernt und der Crimpprozess abgeschlossen.

### Bezugszeichenliste

- 1, 2, 3: Walze
- 11: erster Teil eines C-förmigen Trägers
- 12: zweiter Teil eines C-förmigen Trägers
- 15: Spindel
- 17, 19: durchgehende Öffnung
- 18: Außengewinde
- 21: Druckfeder
- 23, 25: ringförmige Abstützfläche
- 30: Implantat
- 31: distales Ende des Implantats 30
- 40: Kühlmittel
- D1: Abstand zwischen erster Walze 1 und zweiter Walze 2
- D2: Abstand zwischen erster Walze 1 und dritter Walze 3
- D3: Abstand zwischen zweiter Walze 2 und dritter Walze 3

## Patentansprüche

1. Vorrichtung zum Crimpen eines Implantats (30), insbesondere einer intraluminalen Endoprothese, welches zumindest über einen Teil seiner Länge entweder einen komprimierten Zustand oder einen expandierten Zustand einnehmen kann, **dadurch gekennzeichnet, dass** eine Walzenanordnung mit mindestens drei Walzen (1, 2, 3) zum Einspannen des Implantats (30) zwischen diesen vorgesehen ist, wobei mindestens eine Walze (1) der Walzenanordnung in ihrem Abstand zu den jeweils anderen mindestens zwei Walzen (2, 3) derart verringerbar ist, dass ein zwischen den Walzen (1, 2, 3) eingespanntes Implantat (30) wenigstens über einen Teil seiner Länge von dem expandierten Zustand in den komprimierten Zustand überführbar ist.

2. Vorrichtung nach Anspruch 1, **dadurch gekennzeichnet, dass** jede Walze (1, 2, 3) der Walzenanordnung um ihre Längsachse drehbar in einem C-förmigen Träger befestigt ist, wobei die Längsachsen der Walzen (1, 2, 3) vorzugsweise parallel zueinander und besonders bevorzugt parallel zu der Längsachse des zu crimpenden Implantats (30) verlaufen, wenn dieses zwischen den Walzen (1, 2, 3) eingespannt ist.

3. Vorrichtung nach einem der vorhergehenden Ansprüche, **dadurch gekennzeichnet, dass** die Einrichtung zur Verringerung des Abstands der Walzen (1, 2, 3) zueinander seitlich von der Walzenanordnung angeordnet ist.

4. Vorrichtung nach einem der vorhergehenden Ansprüche, **dadurch gekennzeichnet, dass** mindestens eine Walze (1, 2, 3) der Walzenanordnung eine Mantelfläche aufweist, welche Teflon enthält.

5. Vorrichtung nach einem der vorhergehenden Ansprüche, **dadurch gekennzeichnet, dass** die Vorrichtung um das Implantat drehbar ausgebildet ist.

6. Vorrichtung nach einem der vorhergehenden Ansprüche, **dadurch gekennzeichnet, dass** die Vorrichtung in ein Kühlmittel (40) einbringbar ist.

7. Verfahren zum Crimpen eines Implantats (30), insbesondere einer intraluminalen Endoprothese, welches wenigstens über einen Teil seiner Länge entweder einen komprimierten Zustand oder einen expandierten Zustand einnehmen kann, vorzugsweise unter Anwendung einer Vorrichtung nach einem der vorhergehenden Ansprüche, mit den folgenden Schritten:
- Anordnen des zunächst im expandierten Zustand vorliegenden Implantats in einer zwischen mindestens drei Walzen (1, 2, 3) einer Walzenanordnung ausgebildeten Öffnung, wobei das Implantat (30) zwischen den mindestens drei Walzen (1, 2, 3) eingespannt wird, und
- Verringerung des Abstands mindestens einer Walze (1) zu den mindestens zwei anderen Walzen (2, 3) der Walzenanordnung, vorzugsweise schrittweise, derart, dass das zwischen den Walzen (1, 2, 3) eingespannte Implantat (30) wenigstens über einen Teil seiner Länge von dem expandierten Zustand in den komprimierten Zustand überführt wird.

8. Verfahren nach Anspruch 7, **dadurch gekennzeichnet, dass** die gesamte Vorrichtung, welche das Verfahren durchführt, während der Überführung des Implantats (30) in den komprimierten Zustand relativ zu diesem gedreht wird, vorzugsweise schrittweise abwechselnd mit der Verringerung des Abstands der mindestens einen Walze zu den mindestens zwei anderen Walzen.

9. Verfahren nach einem der Ansprüche 7 oder 8, **dadurch gekennzeichnet, dass** die Vorrichtung vor der Überführung des Implantats (30) in den komprimierten Zustand in ein vorzugsweise flüssiges Kühlmittel (40) eingebracht wird und während des Crimpschrittes in dem Kühlmittel (40) verbleibt.

10. Verfahren nach einem der Ansprüche 7 bis 9, **dadurch gekennzeichnet, dass** nach dem Crimpen über den Teil der Länge des Implantats (30), welcher in den komprimierten Zustand überführt wurde, ein Schlauch angeordnet wird.
